(19)

**Europäisches Patentamt**
**European Patent Office**
**Office européen des brevets**

(11)    **EP 4 166 131 A1**

(12)    **EUROPEAN PATENT APPLICATION**

(43) Date of publication:
**19.04.2023  Bulletin 2023/16**

(21) Application number: **21202428.5**

(22) Date of filing: **13.10.2021**

(51) International Patent Classification (IPC):
**A61K 9/127** (2006.01)    **G01N 33/50** (2006.01)
**A61K 45/00** (2006.01)

(52) Cooperative Patent Classification (CPC):
**G01N 33/5076; A61K 45/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Paris Sciences et Lettres**
  **75006 Paris (FR)**
• **Ecole Normale Supérieure**
  **75005 Paris (FR)**
• **Centre national de la recherche scientifique**
  **75016 Paris (FR)**

• **SORBONNE UNIVERSITE**
  **75006 Paris (FR)**
• **Université de Paris**
  **75006 Paris (FR)**

(72) Inventors:
• **THIAM, Abdou Rachid**
  **91200 ATHIS-MONS (FR)**
• **SANTINHO, Alexandre**
  **92000 NANTERRE (FR)**

(74) Representative: **Novagraaf Technologies**
  **Bâtiment O2**
  **2, rue Sarah Bernhardt**
  **CS90017**
  **92665 Asnières-sur-Seine Cedex (FR)**

(54)    **GIANT ORGANELLES RECOVERY AND USE THEREOF**

(57)    The present invention concerns a new method based on the combination of an osmotic swelling to generate stable and functional giant organelles and an adapted cell membrane lysis to isolate and recovery said organelles, and use of said organelles to screen the activity of proteins or exogenous molecules.

FIGURE 4

## Description

## Technical field of the invention

The present invention concerns a new method based on the combination of an osmotic swelling to generate over-micrometric swollen intracellular organelles having an increased size which is generally over 1 $\mu$m (hereinafter referred to as giant organelles (GOs) and an adapted cell membrane lysis to isolate and recover said stable and functional giant organelles (i.e. organelles having an increased size which is generally much larger than 1 $\mu$m), and use of such giant organelles for several applications including the screening of the activity of proteins or exogenous molecules or drugs.

## State of the art

A major goal and limitation in cell biology, biochemistry, drug discovery, pathogenesis, is to know what happens to each organelle upon a given environmental change. It is important to know where proteins are exactly localized, how they get recruited and impact organelle properties, what are their functions. Proteins such as ion channels or enzymes constitute for instance essential pharmacological drug targets. All these limitations could be circumvented if one could touch the organelles and modulate them.

Current methods are often based on the recovery of live cell membrane fragments (hundreds of nanometers sized) or cell fragment recovery followed by a long biochemical analysis which is usually used to determine whether a protein is present or not and at which quantity. No optical visualization is possible. Recent studies have shown that submitting cells to a hypotonic buffer leads to the swelling of organelles, leading to the formation of intracellular organelles of increased size. However in these experiments, the cells remained intact and the organelles were trapped.

Some companies selling cell lysis kit, developed a protocol by which a slight hypotonic medium used with detergents followed by a shear induced on cells at high pressure (>1bar) with macroscopic syringes (>0,5 mm in radius) lead to cell lysis (https://www.thermofisher.com/sn/en/home/life-science/proteinbiology/protein-biology-learning-center/protein-biology-resource-library/pierceprotein-methods/traditional-methods-cell-lysis.html; https://www.sigmaaldrich.com/FR/fr/product/sicigma/mcl1?gclid=Cj0KCQiw0K-HBhDDARIsAFJ6UGgiWEUGjKbual-z q3z21w7hBtnBD z3iEBRTtuqNDZ4NRz rFxdXwaAqM5EALw_wcB). These companies do not form giant organelles during this process: Their swelling protocol is not sufficient to generate stable and functional giant organelles and their shearing process is so strong that the organelles break apart into fragments; generating heterogeneous membrane debris, i.e. nano-fragments of organelles, from one organelle with potential loss of biochemical composition (luminal and membrane proteins can be loosed during the process), activity, functionality and/or membrane biophysical properties.

Thus there is still a need to generate, extract and recover stable and functional swollen organelles, otherwise called giant organelles, from any cells.

## Description of the invention

Therefore the Inventors established a method based on a particular combination of osmotic, physicochemical perturbations and micro-manipulation to isolate and recover stable and functional giant organelles from cells. To this aim the Inventors have developed a method based on both osmotic swelling to generate intracellular organelles having an increased size compared to their original form, otherwise called giant organelles, then generating a plasma membrane tension increase of swollen cells leading to lysis of the plasma membrane and the release of the cell interior, in particular stable and functional giant organelles (i.e. endoplasmic reticulum, mitochondria, nucleus, Golgi apparatus, lysosome, endosome, plasma membrane, etc...). To sum up, in the whole protocol, the strong osmotic shock allows to destabilize the plasma membrane of cells, which then only needs a small mechanical perturbation to break and release the giant organelles.

According to the present invention, the term "giant organelles" (GOs) means organelles having a mean size generally over 1 $\mu$m, from about 1 to 50 $\mu$m, preferably from about 2 to 15 $\mu$m, more preferably from about 3 to 10 $\mu$m, the most preferably of about 5 $\mu$m. Such giant organelles have a surface-to-volume ratio decreased compared to their native form which ranges from about 3 $\mu m^1$ to about 0,06 $\mu m^{-1}$, preferably from about 1,5 $\mu m^{-1}$ to about 0,15 $\mu m^{-1}$, more preferably from about 0,3 $\mu m^{-1}$ to about 1 $\mu m^{-1}$. Such giant organelles have a membrane tension above $10^{-6}$mN/m and below 10mN/m.

The method of the present invention allows to obtain giant organelles, i.e. "macroscopic" swollen organelles isolated from its owing cell, entire or in a large portion [unlike other techniques where only debris or heterogeneous organelles fragments generally of nanometric (about 500 nm size and lower than the micron) are obtained, where proteins initially embedded in organelle membranes could be removed due to the presence of chemical detergents in cell lysis kits]. The giant organelles of the present invention make it now possible to turn toward applications previously unthinkable for industrial players. In addition, contact between such giant organelles can be conserved. Another advantage is the rapid isolation of giant organelles easily imaged with a microscope (e.g. about 3-10 $\mu$m sized), which, for example, allows a fast quantification of drug interaction and/or effect on protein activity. This facilitates the process compared to other techniques from the art which have to concentrate the fragments of organelles and then do some biochemistry quantification.

Such recovered giant organelles are functional, can be labelled, have any protein wanted on them, and can be easily picked and manipulated.

This technology enables the recovery of giant organelles that could be used to screen membrane/luminal proteins activity. In the field of pharmaceutical R&D, this activity can be measured with or without the presence of a drug. It concerns membrane and soluble proteins or enzymes, e.g. ions channels, ribosomes, etc....

More globally, this technology offers for the first time the possibility to map drug-organelle interactions to establish the selectivity/specificity and/or toxicity of drug candidate interacting with proteins localized to a given organelle.

The present invention thus relates to a method for recovery of stable and functional giant or swelled organelles from any cells, said method comprising :

a) Osmotic swelling of cells and organelles into an hypotonic medium with water dilution ranging from about 2X (~150 mOsm) to 1000X (~0.1 mOsm or deionized water), preferably from about 3X to 1000X, thus generating stable and functional intracellular giant (or swollen) organelles for example having a mean size over 1 $\mu$m, ranging from about 1 to 50 $\mu$m, preferably from about 2 to 15 $\mu$m, more preferably from about 3 to 10 $\mu$m, reaching a maximum size of about 10 $\mu$m ; the most preferably of about 5 $\mu$m ;

b) Generating a stretching membrane tension on cells ranging from about $10^{-6}$ mN/m to 30 mN/m, preferably from about 0.05 mN/m to 15 mN/m, and more preferably from about 0.5 mN/m to 10 mN/m for lysis of cell plasma membranes ;

c) Recovery of stable and functional giant organelles in the hypotonic medium.

According to a particular embodiment of the method of the present invention, the giant organelles recovered from step c) can be further put into a less hypotonic medium with water dilution ranging from about 1X to 5X, to generate stable and functional organelles having a mean size decreased from about 5% to 95%, preferentially from about 10% to 70%, more preferentially around 20% compared to giant organelles recovered from step c). These relative size reductions correspond to organelle mean size shifts. Thus, organelles have a size ranging from about 100 nm to 30 $\mu$m, preferably from about 200 nm to 10 $\mu$m, more preferably around 4 $\mu$m. These organelles also have a shift in membrane tensions. The organelle membrane tensions are ranging from about $10^{-7}$ mN/m to 5 mN/m, preferably from about $10^{-6}$mN/m to 0.1 mN/m, more preferably around 0.05 mN/m.

According to a particular embodiment of the method of the present invention, the cells from which the giant organelles are obtained can be cultured on a support or in suspension in bulk, or can be recovered from tissues, organs or organisms. Any type of cells (mammalian, plant or bacteria cells) can be used to generate giant organelles according to the method of the present invention. For example, cells derived from Cos7, Huh, mammalian cells, human cells, tumor cells, obtained from the market, laboratories and hospital patients.

According to a particular embodiment of the method of the present invention, the hypotonic medium which allows to obtain stable and functional giant organelles, is any aqueous solution with a lower osmolarity than the intracellular medium, such as for example, buffer solutions (Diluted DPBS), diluted cell culture medium (DMEM), ionic solutions, salt solutions (e.g. $CaCl_2$ or KCl solutions), water, etc.... The method of the present invention relies on particular conditions for cell swelling with a diluted buffer ranging from about 150 mOsm to 0.1 mOsm, preferably from about 100 mOsm to 1 mOsm, more preferably from about 5 mOsm to 50 mOsm, or is only $H_2O$, for allowing the generation of giant organelles. Finally any solution having an osmolarity about 50% lower (e.g. for dilution of 2X; ~150 mOsm, preferably below 100 mOsm) than the osmolarity of the cell cytoplasm (and initial culture media (~300mOsm)) will be efficient in generating giant organelles, knowing that the cytoplasm osmolarity of cells are all between about 285 mOsm and 315 mOsm (50% lower is about 150 mOsm). Thus for example, for Cos7 cells, the cytoplasm osmolarity is around 300 mOsm, meaning that all kind of aqueous solutions with an osmolarity below 150 mOsm, preferably below 100 mOsm, more preferably below about 50 mOsm, can be used to generate giant organelles. For example, the hypotonic medium in step a) has a ratio of $H_2O$ to any culture medium type (or ionic aqueous solutions with physiological osmolarity) above 1:1, preferably between 2:1 and 99:1, and more preferably between 4:1 and 59 :1. This manipulation enables to apply an adequate non-destructive fast osmotic swelling to generate stable giant organelles with preserved integrity and functionality. These media can contain protease inhibitors to prevent protein degradation.

According to a particular embodiment of the method of the present invention, prior to the generation of giant organelles, cells may be treated with molecules to prevent protein degradation, modulate biochemical reactions on organelles, etc... so that giant organelles can bear specific biochemical properties. The metabolic conditions and the architecture of cells and its organelles can thus be modified before step b), by chemicals and/or by modifying the expression level of proteins impacting the architecture of cells and/or their organelles and/or the metabolic conditions. Indeed prior to the osmotic swelling, cells can be treated with chemicals or can be modified to overexpress or repress the level of some proteins that impact the architecture of the cell and its organelles - notably the surface-to-volume ratio and the relative positioning of the organelles with each other - and the metabolic conditions. These manipulations enable controlling the size of the recovered giant organelles and their contact: tuning the surface-to-volume ratio, prior to the osmotic swelling, impacting the future recovered giant organelle size, with adapted osmotic swelling. For example, prior to inducing the osmotic swelling, the size of the future giant organelles can be modulated (1) by altering the expression levels of proteins impacting organelle shape, (2) by treating cells with molecules that alter the cytoskeleton, organelle contacts, (3) by altering cellular pathways impacting organelles number and shape, surface, (4) any treatment mediate change in organelle surface-to-volume ratio and inter-organelle contact. For example, overexpressing climp63 prior to the generation and recovery of giant organelles leads to larger giant organelles emanating from the endoplasmic reticulum. In the same way, overexpressing Mfn2 prior to the generation and recovery of giant organelles leads to larger giant organelles emanating from the mitochondria. Pretreating cells with nocodazole (or Latrunculin A) between 1 and 90min before swelling allows the formation of bigger giant organelles from the ER. Adding rapamycin between 12 to 24 hours before swelling formation allows to form bigger giant organelles from endosome, lysosome, autolysosome and multivesicular body. Adding bafylomicin before swelling and extraction also allows to obtain more giant organelles coming from autophagosomes.

According to a particular embodiment of the method of the present invention, step b) is performed and creates a stretching membrane tension (bilayer tension) on cells ranging from about $10^{-6}$ mN/m to 30 mN/m, preferably from about 0.05 mN/m to 15 mN/m, and more preferably from about 0.5 mN/m to 10 mN/m so as to break the plasma membrane while preserving the structure of giant organelles (i.e. there is no fragmentation of organelles into debris lower than the micron with losses of components and activities), by using: mechanical force (e.g. suction pressure, stretching, shearing or acoustic wave), chemical agents and/or detergents, electric field, or laser (light) excitation. For example, the mechanical force is applied with a micro-pipette (radius from about 0,5 to 20 $\mu$m) with a suction pressure (generally ranging from about 0.1 to 600 mbar and more preferably from about 5 to 200 mbar where previous techniques use pressure larger than 1 bar) that creates the expected stretching membrane tension. For example, after cell swelling and intracellular giant organelles formation according to step a) of the method of the present invention, acoustic waves ranging in the ultrasonic range (preferably from about 16 Khz to 1000 Khz, more preferably from about 20 Khz to 400 Khz) during few seconds allows to create the expected stretching membrane tension that leads to the lyse of plasma membrane and giant organelles recovery (see example section). For example, after cell swelling and intracellular giant organelles formation according to step a) of the method of the present invention, chemicals working as detergents can be added in a control manner to create the expected stretching membrane tension that leads to the lyse of plasma membrane and giant organelles recovery, such as carboxylic acid (with concentrations sufficient to generate plasma membrane pore stabilization), Triton X, Tween, SDS, Brij, Octyl Glucoside, octyl thioglucoside, CHAPS, CHAPSO and others. Using detergents modifies the properties of the giant organelles (see example section).

According to the method of the present invention, giant organelles can be recovered without any labeling and sorted out later by using several non-invasive methods including optical, morphological, mechanical approaches. However according to a particular embodiment of the method of the present invention, the cells of step a) can comprise cells transfected with at least one organelle protein marker or receiving molecules reporting for organelles to facilitate organelle identification. For example, the cells are transfected with at least one organelle protein tagged with a fluorescent or chemical marker used before or after swelling. For example, KDEL marks the ER lumen, Tom20 marks mitochondria, LC3B marks autophagosomes, Lamp1 marks lysosomes and Golgi7 marks the Golgi apparatus.

The present invention also relates to giant organelles obtained by a method of the present invention, characterized in that said giant organelles have a mean size generally over 1 $\mu$m, ranging from about 1 to 50 $\mu$m, preferably from about 2 to 15 $\mu$m, more preferably from about 3 to 10 $\mu$m, reaching a maximum size of about 10 $\mu$m ; the most preferably of about 5 $\mu$m.

Recovered giant organelles are swollen, meaning that their surface-to-volume ratio is decreased compared to their native form. This ratio ranges from about 3 $\mu$m$^{-1}$ to 0,06 $\mu$m$^{-1}$, preferably from about 1,5 $\mu$m$^{-1}$ to 0,15 $\mu$m$^{-1}$, more preferably from about 0,3 $\mu$m$^{-1}$ to 1 $\mu$m$^{-1}$. Such giant organelles have a plasma membrane tension ranging from about $10^{-6}$ mN/m to 10mN/m. The giant organelles of the present invention can be recovered isolated or in contact with at least one other giant organelle of the same or different type. The giant organelles of the present invention can be stored, and later delivered for research purposes. For example, such organelles are chosen from the group including the endoplasmic reticulum, mitochondria, lysosome, nucleus, Golgi apparatus, lipid droplets, vacuole, chloroplaste, autophagosome, autolysosomes, endosomes, plasma membranes, peroxisome, multivesicular bodies.

The present invention also relates to a method for screening the activity of a molecule of interest, said method comprising :

a) Contacting at least one type of giant organelles of the present invention with the molecule of interest;
b) Measuring the modulation of the activity of the molecule of interest through its interaction with the flux of said at least one type of giant organelles.
c) Optionally comparing the activity measured in step b) to the initial activity of the molecule of interest before any contact.

The method for screening of the present invention thus enables measuring the capacity of a molecule of interest to interact with at least one protein on a giant organelle, for example screening the efficacy, selectivity, specificity, toxicity, biodisponibility, permeability of any drug candidates with at least one protein localized on giant organelles obtained through the present invention. Moreover, the method of the present invention can also enable, for example, to modulate the communication of contact between giant organelles, to identify molecules targeting the giant organelles, to test toxicity of molecules blocking the activity of giant organelles. This will thus enable precision medicine and early diagnoses realized on giant organelles directly extracted from healthy / sick patient.

The present invention also relates to the use of at least one giant organelle according to the present invention, for screening the activity of molecules of interest. The interaction or the impact of compounds such as drugs, proteins (e.g. involved in a disorder), or ions on giant organelles of the present invention and the contact and communication between them can thus be assessed before the generation and recovery of giant organelles. The giant organelles of the present invention can thus be used for studying biochemical reactions, or for disease's diagnosis for example by defining patterns of diseases from sick patients and comparing them with healthy patients.

The present invention also relates to the use of at least one giant organelle according to the present invention as a carrier, thus enabling encapsulation or sequestration of a molecule of interest, e.g. an exogeneous molecule or a molecule/protein expressed by the cell prior to the generation of the giant organelles, for drug delivery applications.

**Brief description of the figures**

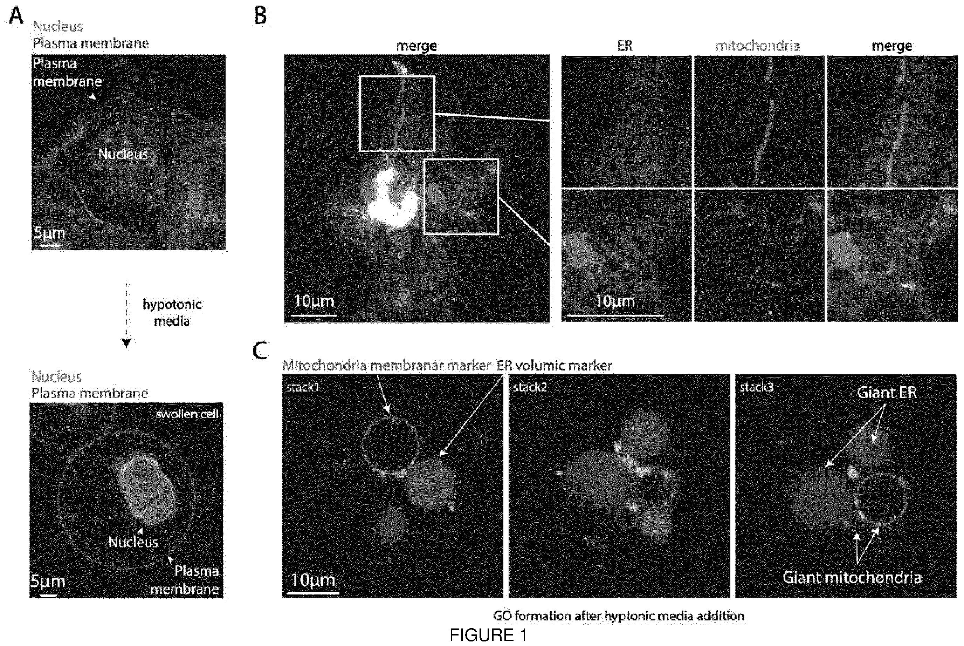

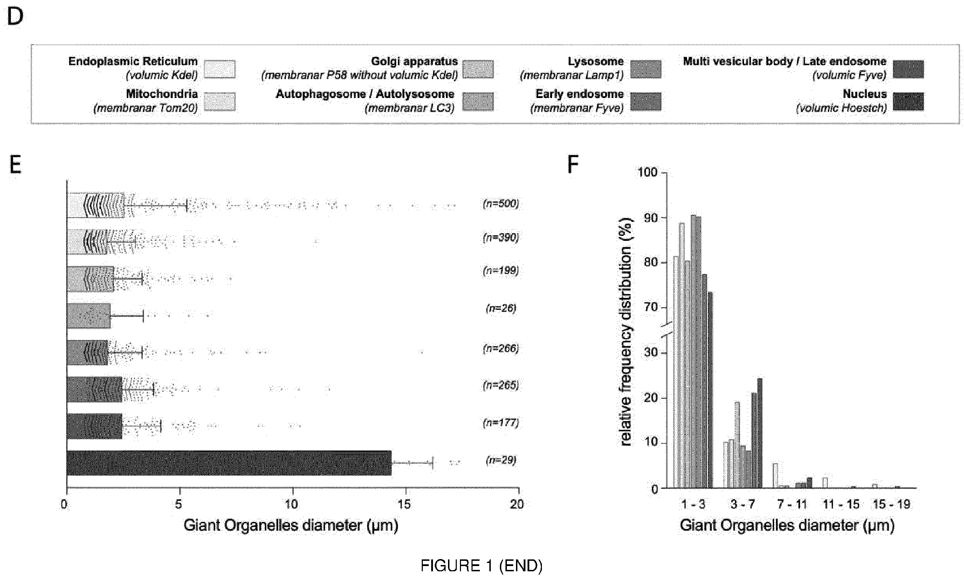

Figure 1 represents (**A**). Top. Confocal microscopy snap of a Cos7 cell over-expressing a fluorescent plasma membrane protein (GPI-mcherry) where the nucleus was probed with Hoetsch. **Bottom.** Confocal microscopy snap of a swollen Cos7 cell with same transfections as above. (**B**). Confocal microscopy snap of a Cos7 cell over-expressing fluorescent both volumic ER protein (RFP-Kdel) and a membranar mitochondria protein (Mfn2-YFP). (**C**). Confocal microscopy snap of a swollen Cos7 cell over-expressing fluorescent both volumic ER protein (RFP-Kdel) and a membranar mitochondria protein (Mfn2-YFP). These snaps clearly showed the formation of giant organelles (GO): here Giant ER (GER) and Giant Mitochondria (GM) are visualized. (**D**). Rectangular panel to differentiate each type of GOs was generated: ER, mitochondria, Golgi apparatus, autophagosome/autolysosome, lysosome, early endosome, multivesicular body/late endosome and nucleus. (E). Plot of the average GO diameter for each type of organelles. Related to Figure 1D. Mean +/-standard deviation; n report the number of organelles analyzed for each condition. (**F**). Plot of the relative frequency distribution of GO diameter depending on organelles type. Related to Figure 1D-E.

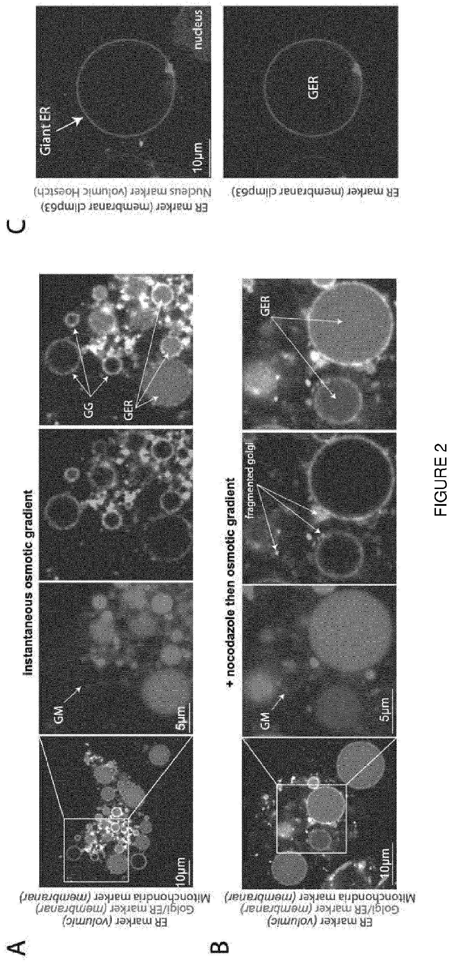

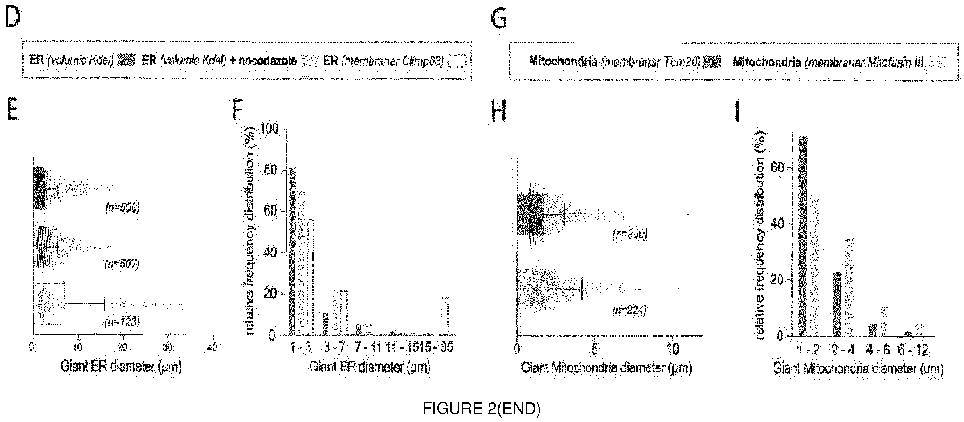

Figure 2 represents (**A**). Confocal microscopy snap of a swollen Cos7 cell over-expressing following fluorescent proteins: RFP-Kdel (volumic marker of GERs), mcherry-TOMM20-N (membranar marker of GMs) and P58-GFP reporting for GERs (P58 signal with RFP-Kdel) and Giant Golgi (GGs) (P58 signal without RFP-Kdel). (**B**). Confocal microscopy snap of a swollen Cos7 cell over expressing same proteins as in figure 2A. In this case, cells were pre-treated with nocodazole 1h before swelling. Formation of huge ER membrane thanks to overexpression of the mCherry-climp63 protein (**D**). Rectangular panel showing the different marker used to differentiate three different conditions tested in figure 2A,B,C. (**E**). Plot of the average GER diameter for each condition of panel 2D. Mean +/standard deviation; n report the number of organelles analyzed for each condition. (**F**). Plot of the relative frequency distribution of GER diameter for each condition of panel 2D. (**G**). Rectangular panel defining two different types of GMs, *i.e.* extracted from cell over-expressing mcherry-TOMM20-N or Mfn2-YFP. (**H**). Plot of the average GM diameter for both conditions of figure 2G. Mean +/standard deviation; n report the number of organelles analyzed for each condition. (**I**). Plot of the relative frequency distribution of GM diameter (related to figure 2G-H).

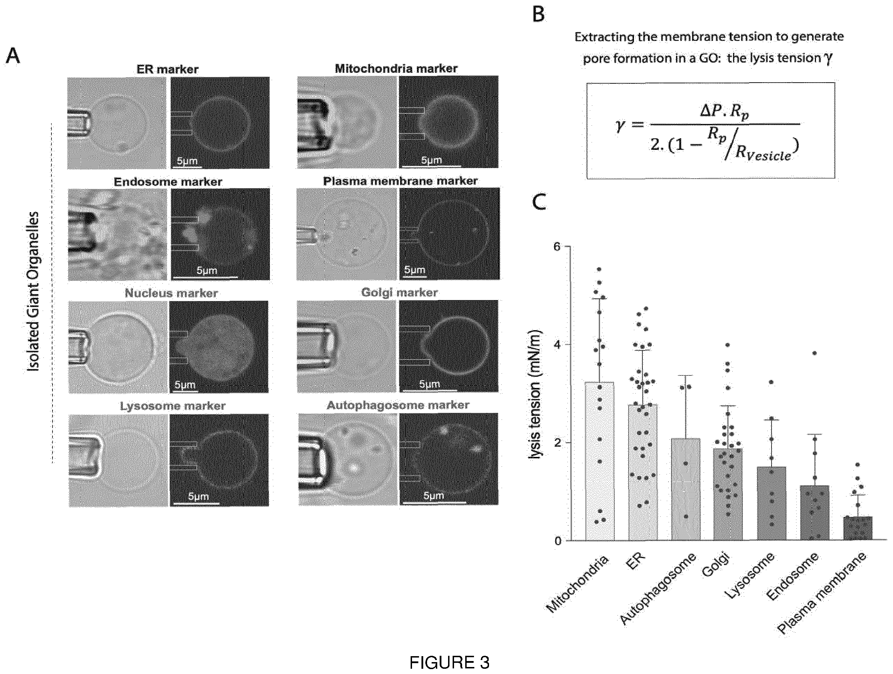

Figure 3 represents (**A**). Mathematical formula derived from Laplace law to compute the membrane tension of a GO. The equation parameters were defined in figure 3A. ΔP corresponds to the aspiration pressure imposed into the micropipette. (**B**). Confocal microscopy of recovered GOs. (Fluorescence reporter: ER: RFP-Kdel; Mitochondria: mcherry-TOMM20-N; Endosome: pmCherry-2X-FYVE; Plasma membrane: GPI_2xmCherry; Golgi Apparatus: P58-GFP; Lysosome: pMRXIP Lamp1-Venus; Autophagosome: LC3-EGFP; Nucleus: Hoestch 33342). (**C**). Measurement of lysis tension of isolated GOs.

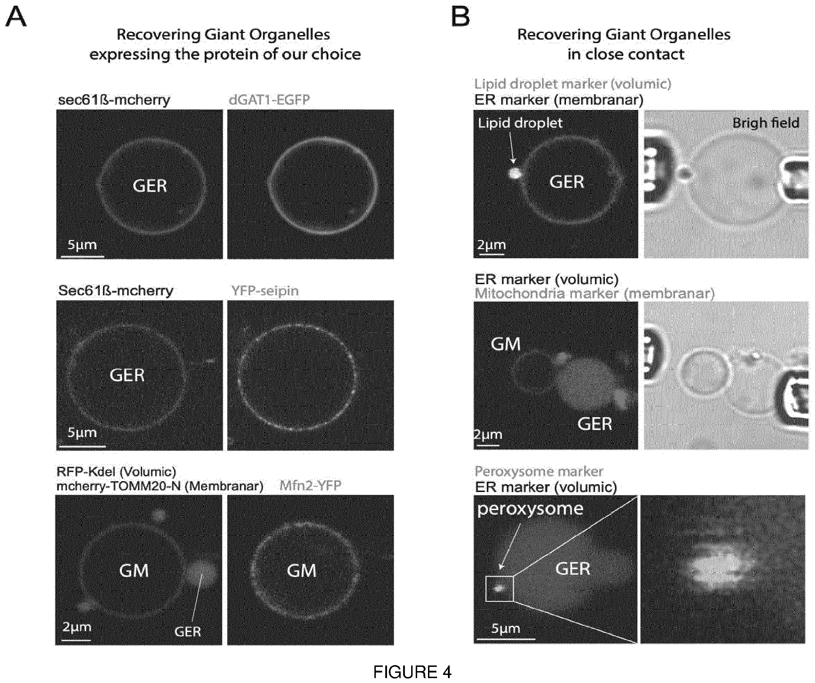

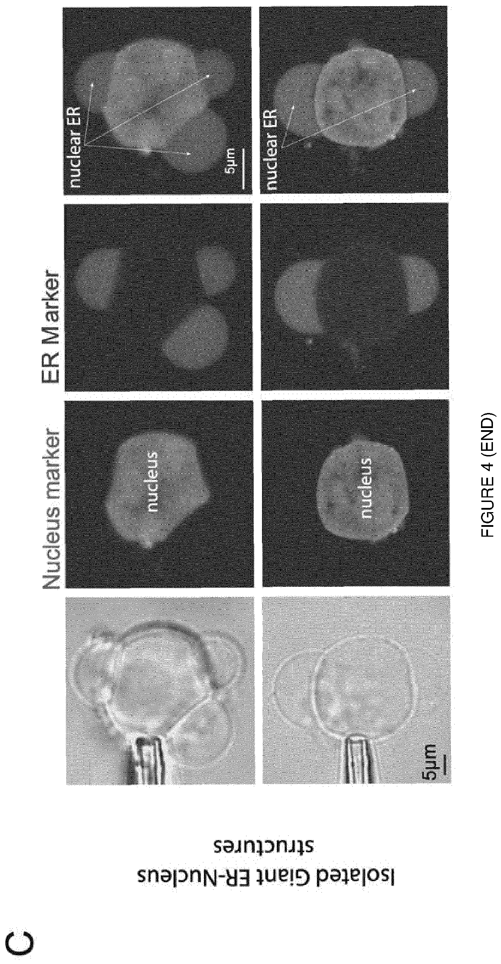

Figure 4 represents (**A**). Confocal microscopy snaps of recovered GOs expressing different proteins of our choice: **Top.** GER covered by YFP-seipin and sec61β-mCherry. **Middle.** GER covered by dGAT1 -EGFP and sec61β-mCherry. **Bottom.** GM covered by Mfn2-YFP and mcherry-TOMM20-N. (**B**). Confocal microscopy snaps where GIANT ORGANELLEs in contact are recovered: **Top.** A GER (sec61β-mcherry) contacting a lipid droplet (tagged with Bodipy-FL) is shown. **Middle.** A GER (RFP-Kdel) contacting a GM (mcherry-TOMM20-N) is shown. **Bot-**

**tom.** A GER (RFP-Kdel) contacting a peroxisome. (**C**). Confocal microscopy snaps of nucleus (Hoestch 33342) recovered in contact with nuclear ER (RFP-Kdel) portion of revealing large contacting area between these organelles.

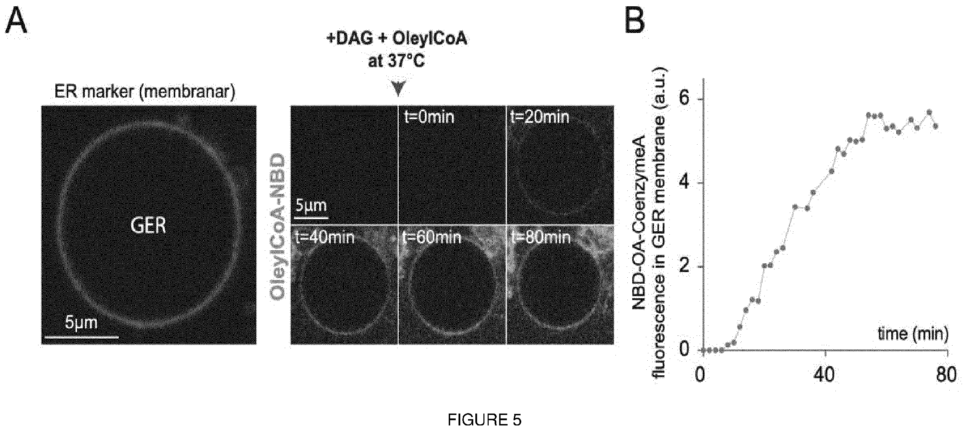

Figure 5 represents (**A**). Snap of an isolated GER (sec61β-mcherry) fed with OleylCoA (complemented with NBD-OleylCoA) and DiAcylGlycerol (DAG) at 37°C. As the organelle get the dGAT1 enzyme (Related to figure 4A, middle), we triglycerides synthesis was visualized in the GER membrane among time thanks to the incorporation of OleylCoA-NBD signal in the GER. (**B**). Plot of the fluorescence signal of OleylCoA-NBD in the GER during the feeding experiment (Related to figure 5A).

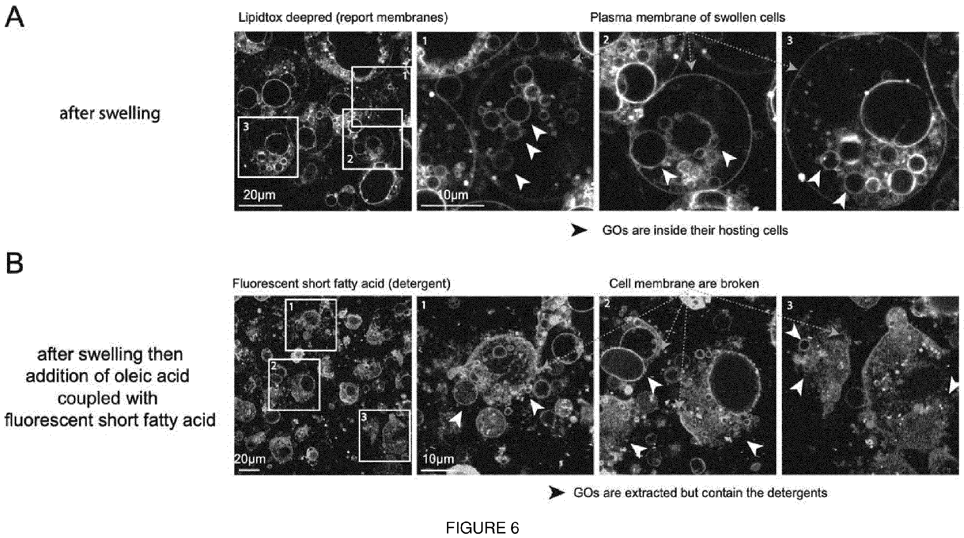

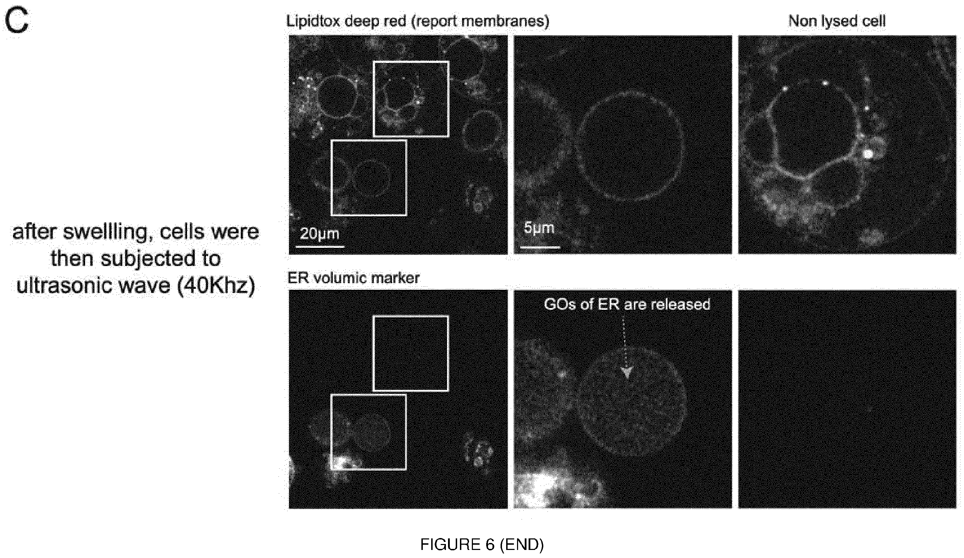

Figure 6 represents (**A**). Confocal microscopy snap of Cos7 cells after the swelling step. Cells are entire and their plasma membrane are intact. (**B**). Confocal microscopy snap of Cos7 cells after the swelling step and the addition of detergent (Oleic acid) combine with fluorescent fatty acids. The addition of detergents after GO formation allow the release of GOs in the bulk thanks to the breakage of plasma membrane. GO membrane also incorporate the detergents, which can modify their properties. (**C**). Confocal microscopy snap of Cos7 cells after the swelling step and a step where the cells were subjected to ultrasonication (acoustic wave of 40Khz). This acoustic field allows the release of GOs (ER, cells over expressed RFP-Kdel) in the bulk.

## EXAMPLES

### EXAMPLE 1 : METHOD FOR GENERATING GIANT ORGANELLES AND COLLECTING THE SAME: USING MECHANICAL FORCE (SUCTION PRESSURE) AFTER SWELLING

### Cell culture

Cos7 mammalian cells were maintained in Dulbecco's Modified Eagle Medium (DMEM) supplemented with 10% heat inactivated FBS and 1% penicillin-streptomycin. Before the GOs recovery protocol, cells were cultivated 48h in DMEM media at 37°C with 5% $CO_2$. Moreover, cells were transfected 24h with the indicated plasmids to probe different organelles before extraction and recovery. Cells were cultured left adhering in Matek dishes, pre-treated or not with adhesion agents.

### Cell transfection reporting for giant organelles

Cells were transfected with different plasmids fused with fluorescent protein constructs (e.g., eGFP or mCherry) 24h before the giant organelles extraction and recovery. These plasmids serve to express proteins reporting for different organelles. *Kdel* and Sec61β plasmids were used to identify the endoplasmic reticulum, *Tom20* and/or *MitofusinII* plasmids to identify mitochondria, *GP58* or *Kde* for the Golgi, *FYVE* for endosomes, *LC3* for autophagosome and *Lamp1* for the lysosomes.

### Giant Organelle formation and size distribution

For Giant Organelle formation, before cells were confluent, the cell culture media was diluted with $H_2O$, pH 7.4, at 37°C and 5% $CO_2$. See the swollen cells before giant organelles extraction after osmosis (**Figure 1C**). The osmolarity gradient of the swelling solution was controlled and was adjusted depending on the cell type. After osmosis, the cell solution was injected in clear hypotonic media on a glass plate, pre-treated with Bovine Serum Albumine (BSA) 10% v/v. Snaps were then taken on entire swollen cells with different organelles protein markers to visualize giant organelles. The diameter of each GOs was measured on each stacks of these swollen cells to report for size distribution of GOs. **(Figure 1E-F).** As we are interested on over-micrometric organelles, we only considered organelles over 0,75 μm in size distribution measurements. We defined giant organelles when swollen organelles exhibit diameter bigger than 1 μm.

### Size control of Giant Organelles

To control the GOs formation, the swelling of organelles was carried out thanks to the initial hypotonic media **(Figure 2A).** Adding some chemical reagent allows us to control even more the distribution size of organelles.

To increase the size of Giant Endoplasmic Reticulum (GER), we added nocodazole at 2.5μM in the culture media one hour before extraction **(Figure 2B).** The cells were also transfected with a ER membrane shaping protein: *Climp63* **(Figure 2C).** The protocol with nocodazole allowed to form a higher proportion of Giant ER than without **(Figure 2D-F).** Moreover, over-expressing the protein *Climp63* in cells allows the formation of huge GER (15μm-35μm) **(Figure 2D-F).**

Following this idea of over-expressing membrane shaping proteins, the size of Giant Mitochondria (GM) was also controlled. The *mitofusinII* protein was overexpressed instead of the *TOM20* mitochondria membrane proteins (Figure 2H *vs* 2I). Over-expressing the *mitofusinII* protein allowed to form bigger GMs.

### (Figure 2G-I).

### Controlling the release of giant organelles thanks to pore opening in plasma membrane.

After osmosis, cells were micro-manipulated under confocal microscope. **(data not shown).** Giant organelles were observed in the culture media. The giant organelles were released thanks to pore opening in the plasma membrane **(data not shown).** To quantify the surface energy to provide on the plasma membrane to generate the opening of a pore and giant organelles releasing, the membrane lysis tension was measured, *i.e.* the membrane tension when a pore opens **(Figure 3A)**. The membrane lysis tension of all giant organelles recovered and isolated was also measured **(Figure 3B)**. With our protocol conditions, the plasma membrane lysis tension was found to be lower than all other giant organelles lysis tension **(Figure 3C)**.

**Giant organelles lysis tension measurements:**

To determine an estimation of the bilayer lysis tension of giant organelles, the micropipette aspiration technique was used. Micropipette radius were around $1 \mu$m. Thanks to a slight aspiration, a bilayer tongue was sucked into the micropipette. The aspiration was then increased at an approximate rate of 10 mbar/min, causing a proportional increase in the bilayer surface tension (see giant organelles aspiration tension measurements). At a certain tension, the giant organelles ruptured because of a pore opening in its membrane. Therefore the measured lysis tension was taken as the higher tension reached just before bilayer rupture. Using Laplace's law, and the measurement of the pipette inner radius ($R_p$), Giant Organelle radius ($R_{\text{Giant Organelle}}$), and suction pressure $\Delta P$, the surface tension $\gamma$ of the interface was calculated:

$$\gamma = \frac{\Delta P . R_p}{2 . (1 - R_p / R_{Vesicle})}$$

The suction was carried out using a syringe. The resulting pressure was measured with a pressure transducer (DP103; Validyne Engineering, North-ridge, CA), the output voltage of which was monitored with a digital volt- meter. The pressure transducer (range 55 kPa) was calibrated before the experiments.

**Giant organelles expressing a protein interest**

One of the great potential of the method of the present invention was the recovery of a giant organelle over-expressing a chosen protein, or few chosen proteins **(Figure 4A)**

**Giant organelles in contact are isolated**

Contact between two different giant organelles were also isolated (such as GER-GM and GER-lipid droplets) **(Figure 4B)**. Particular systems were also isolated like the nucleus-ER system **(Figure 4C)**.

**EXAMPLE 2 : METHOD FOR GENERATING GIANT ORGANELLES AND COLLECTING THE SAME: USING DETERGENTS AFTER SWELLING**

**Giant organelles formation: same protocol as in Example 1**

**Controlling the release of giant organelles thanks to pore opening in plasma membrane by adding chemicals or detergents. (Figure 6B)**

After cell culture, transfection and osmosis, a chemical which act as a detergent was added. An oleic acid solution (solubilized in $H_2O$ with 10% BSA) was added in the swollen cell solution at $400 \mu$M (Bodipy-$C^{12}$ fluorescent probe was added to visualize the incorporation of oleic acid in membranes). After few minutes cells were imaged. Giant organelles were extracted and visualized outside the cells which exhibit plasma membrane lysis.

Adding, these detergents on swollen cells with giant organelles inside allow the extraction and recovery of giant organelles. As expected, the membrane properties of giant organelles are modified due to accumulation of detergents inside their membrane.

**EXAMPLE 3 : METHOD FOR GENERATING GIANT ORGANELLES AND COLLECTING THE SAME: USING ULTRASONIC WAVE AFTER SWELLING (Figure 6C)**

**Giant organelles formation: same protocol as in Example 1**

**Controlling the release of giant organelles subjecting the cells to an acoustic field**

After cell culture, transfection and osmosis, the swollen cell solution was subjected to an acoustic field in a bath sonicator during few seconds. A 40Khz ultrasonic frequency was used to lyse the cells and release giant organelles in the bulk.

**EXAMPLE 4: GIANT ORGANELLES FUNCTIONALITY**

At this step the functionality of recovered giant organelles was studied regarding both assimilation and transformation of oleyl-CoA of giant organelles.

**Triglyceride synthesis. Oil synthesis in Giant Endoplasmic Reticulum (GER)**

**Triglyceride synthesis** : A mixture of diolein at 8mM, Oleyl-CoA at 2mM (+NBD-Oleyl-CoA at $20 \mu$M) complexed with BSA (0,5%) in hypotonic culture media (DMEM:$H_2O$ 5:95) was prepared before the experiment. The beginning of the oil synthesis protocol in GER was the same than in the "formation an extraction of the giant organelles" section. After extraction, giant organelles were incubated during 30minutes at 37°C and 5% $CO_2$ with the addition of 50μL of the feeding mixture. Finally, the final concentrations were 200μM, 50μM and 0,5μM respectively for the diolein, Oleyl-CoA and NBD-Oleyl-CoA in 2mL of hypotonic culture media. For **Figure 5A,** the incorporation of oleic acid in GER was monitored thanks to the fluorescence signal of NBD-Oleyl-CoA in the GER membrane.

**Equipment**

All micrographs were made on a Carl ZEISS LSM 800. Glass coverslips were from Menzel Glaser (24 36 mm #0). Micropipettes were made from capillaries (1.0 OD 0.58 ID 150 Lmm 30-0017 GC100-15b; Harvard Apparatus) with a micropipette puller (model P-2000; Sutter Instruments). Micromanipulation was performed with TransferMan 4r (Eppendorf). Pressure measurement unit (DP103) was provided by Validyne Engineering.

**Viability measurements**

Under conditions where cells were swollen and subjected to a mechanical forces, the membrane lysis tension of each organelle and the plasma membrane of resting cells were measured. All the giant organelles had a membrane lysis tension higher than the plasma membrane.

**Claims**

1. Method for recovery of stable and functional giant organelles from cells, said method comprising :

   a) Osmotic swelling of cells and intracellular organelles into an hypotonic medium ranging from a dilution of 2X to 1000X ;
   b) Generating a stretching membrane tension on cells ranging from $10^{-6}$ mN/m to 30 mN/m for lysis of cell plasma membranes ;
   c) Recovery of stable and functional giant organelles into the hypotonic medium.

2. Method according to claim 1, wherein the cells from which the giant organelles are recovered are cultured on a support or in bulk, or are recovered from tissues, organs or organisms.

3. Method according to either of claim 1 or 2, wherein the hypotonic medium in step a) ranges from a dilution of 3X to 1000X.

4. Method according to any of claims 1 to 3, wherein the hypotonic medium in step a) has an osmolarity about 50% lower than the osmolarity of the cell cytoplasm, preferably an osmolarity below 100 mOsm.

5. Method according to any of claim 1 to 4, wherein the hypotonic medium in step a) has a ratio of $H_2O$ to culture medium above 1:1.

6. Method according to any of claims 1 to 5, wherein step a) generates stable and functional intracellular giant organelles having a mean size from 1 to 50 μm.

7. Method according to any of claims 1 to 6, wherein step b) is carried out using mechanical force, chemical agents, detergents, electric field, and laser excitation.

8. Method according to any of claims 1 to 7, wherein the cells of step

   a) are transfected with at least one organelle protein marker or receiving molecules reporting for organelles.

9. Giant organelles obtained by a method according to any of claims 1 to 8, **characterized in that** said organelles have a mean size ranging from 1 to 50 μm.

10. Giant organelles according to claim 9, further **characterized in that** said giant organelles have a membrane tension ranging from $10^{-6}$ mN/m to 10mN/m.

11. Giant organelles according to either of claim 9 or 10, chosen from the group consisting of endoplasmic reticulum, mitochondria, lysosome, nucleus, ribosome, Golgi apparatus, centrosome, lipid droplets, vacuole, chloroplaste, autophagosome, autolysosomes, endosomes, plasma membranes, peroxisome, multivesicular bodies.

12. Method for screening the activity of a molecule of interest, said method comprising :

   a) Contacting a flux of at least one type of giant organelle according to either of claim 9 or 10 with the molecule of interest;
   b) Measuring the activity of the molecule of interest through its interaction with the flux of said at least one type of giant organelles
   c) Optionally comparing the activity measured in step b) to the initial activity of the molecule of interest before any contact.

13. Use of at least one giant organelle according to either of claim 9 or 10, for screening the activity of a molecule of interest.

FIGURE 1

FIGURE 1 (END)

FIGURE 2

FIGURE 2(END)

**A**

Isolated Giant Organelles

ER marker

Mitochondria marker

Endosome marker

Plasma membrane marker

Nucleus marker

Golgi marker

Lysosome marker

Autophagosome marker

**B**

Extracting the membrane tension to generate pore formation in a GO: the lysis tension $\gamma$

$$\gamma = \frac{\Delta P . R_p}{2 . \left(1 - \dfrac{R_p}{R_{Vesicle}}\right)}$$

**C**

FIGURE 3

A — Recovering Giant Organelles expressing the protein of our choice

sec61ß-mcherry | dGAT1-EGFP

GER

5µm

Sec61ß-mcherry | YFP-seipin

GER

5µm

RFP-Kdel (Volumic)
mcherry-TOMM20-N (Membranar) | Mfn2-YFP

GM

GER

2µm

GM

B — Recovering Giant Organelles in close contact

Lipid droplet marker (volumic)
ER marker (membranar)

Lipid droplet

GER

2µm

Brigh field

ER marker (volumic)
Mitochondria marker (membranar)

GM

GER

2µm

Peroxysome marker
ER marker (volumic)

peroxysome

GER

5µm

FIGURE 4

FIGURE 4 (END)

FIGURE 5

FIGURE 6

C

Lipidtox deep red (report membranes)                    Non lysed cell

after swellling, cells were
then subjected to
ultrasonic wave (40Khz)

20μm                          5μm

ER volumic marker

GOs of ER are released

FIGURE 6 (END)

Europäisches
Patentamt

European
Patent Office

Office européen
des brevets

# EUROPEAN SEARCH REPORT

Application Number

EP 21 20 2428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RIEDER S E ET AL: "Overview of subcellular Fractionation Procedures for the Yeast Saccharamyces cerevisiae", CURRENT PROTOCOLS IN CELL BIOLOGY, JOHN WILEY & SONS INC, US, vol. Unit 3.7, no. Suppl.7, 1 July 2000 (2000-07-01), pages 3.7.1-3.7.25, XP008084705, ISSN: 1934-2500 | 9-13 | INV. A61K9/127 G01N33/50 A61K45/00 |
| Y | * page 3.7.5 – right-hand column, last paragraph * <br> * page 3.7.15 – left-hand column, paragraph 1 * <br> * page 3.7.7 – right-hand column, paragraph 2 * <br> * page 3.7.14 – right-hand column, last paragraph * <br> ----- | 1-13 | |
| X | JAISWAL ASTHA ET AL: "Improved spatial resolution by induced live cell and organelle swelling in hypotonic solutions", SCIENTIFIC REPORTS, vol. 9, no. 1, 9 September 2019 (2019-09-09), XP055912692, DOI: 10.1038/s41598-019-49408-2 Retrieved from the Internet: URL:http://www.nature.com/articles/s41598-019-49408-2> | 9-11 | |
| Y | * Material and Methods * <br> * abstract * <br> ----- <br> -/-- | 1-13 | |

TECHNICAL FIELDS
SEARCHED (IPC)

A61K
G01N

The present search report has been drawn up for all claims

| Place of search | Date of completion of the search | Examiner |
|---|---|---|
| Munich | 21 April 2022 | Bayer, Martin |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
..................................................................................
& : member of the same patent family, corresponding
   document

EPO FORM 1503 03.82 (P04C01)

Europäisches
Patentamt

European
Patent Office

Office européen
des brevets

## EUROPEAN SEARCH REPORT

Application Number

EP 21 20 2428

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LE ROUX ANABEL-LISE ET AL: "The plasma membrane as a mechanochemical transducer", PHILOSOPHICAL TRANSACTIONS. ROYAL SOCIETY OF LONDON. B: BIOLOGICAL SCIENCES., vol. 374, no. 1779, 19 August 2019 (2019-08-19), page 20180221, XP055912767, GB ISSN: 0962-8436, DOI: 10.1098/rstb.2018.0221 Retrieved from the Internet: URL:https://royalsocietypublishing.org/doi/pdf/10.1098/rstb.2018.0221> | 9-11 | |
| Y | * C) Tensile Stress; page 1 * | 1-13 | |
| X | DAI JIANWU ET AL: "Membrane Tension in Swelling and Shrinking Molluscan Neurons", THE JOURNAL OF NEUROSCIENCE, vol. 18, no. 17, 1 September 1998 (1998-09-01), pages 6681-6692, XP055912815, US ISSN: 0270-6474, DOI: 10.1523/JNEUROSCI.18-17-06681.1998 Retrieved from the Internet: URL:https://www.jneurosci.org/content/jneuro/18/17/6681.full.pdf> | 9-11 | TECHNICAL FIELDS SEARCHED (IPC) |
| Y | * page 6684 - right-hand column, paragraph 2 * | 1-13 | |

-----

-----

-/--

The present search report has been drawn up for all claims

| Place of search | Date of completion of the search | Examiner |
|---|---|---|
| Munich | 21 April 2022 | Bayer, Martin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

Europäisches
Patentamt

European
Patent Office

Office européen
des brevets

## EUROPEAN SEARCH REPORT

Application Number

EP 21 20 2428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KING CHRISTOPHER ET AL: "ER membranes exhibit phase behavior at sites of organelle contact", PROCEEDINGS OF THE NATIONAL ACADEMY OF SCIENCES, vol. 117, no. 13, 16 March 2020 (2020-03-16), pages 7225-7235, XP055912644, ISSN: 0027-8424, DOI: 10.1073/pnas.1910854117 Retrieved from the Internet: URL:https://www.pnas.org/doi/pdf/10.1073/pnas.1910854117> | 9-13 | |
| Y | * page 7225 * <br> * page 7226 * <br> * page 7234 * | 1-13 | |
| X | SANTINHO ALEXANDRE ET AL: "Membrane Curvature Catalyzes Lipid Droplet Assembly", CURRENT BIOLOGY, CURRENT SCIENCE, GB, vol. 30, no. 13, 21 May 2020 (2020-05-21), page 2481, XP086208034, ISSN: 0960-9822, DOI: 10.1016/J.CUB.2020.04.066 [retrieved on 2020-05-21] | 9-11 | |
| Y | * figures 1-5 * <br> * Large ER-derived Intra-cellular vesicle experiments; page e3 * | 1-13 | TECHNICAL FIELDS SEARCHED (IPC) |
| X | WO 2019/113512 A1 (FLAGSHIP PIONEERING INNOVATIONS V INC [US]) 13 June 2019 (2019-06-13) | 9-11 | |
| Y | * abstract * <br> * example 3 * | 1-13 | |

The present search report has been drawn up for all claims

| Place of search | Date of completion of the search | Examiner |
|---|---|---|
| Munich | 21 April 2022 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 2428

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2022

| Patent document cited in search report | | Publication date | Patent family member(s) | | Publication date |
|---|---|---|---|---|---|
| WO 2019113512 | A1 | 13-06-2019 | CA | 3083286 A1 | 13-06-2019 |
| | | | CN | 111655292 A | 11-09-2020 |
| | | | EP | 3720502 A1 | 14-10-2020 |
| | | | US | 2021187018 A1 | 24-06-2021 |
| | | | WO | 2019113512 A1 | 13-06-2019 |

EPO FORM P0459

For more details about this annex : see Official Journal of the European Patent Office, No. 12/82